# EUROPEAN PATENT SPECIFICATION

(11) **EP 0 363 879 B1**
(45) Date of publication and mention of the grant of the patent: **16.06.1993**
(21) Application number: 89118741.1
(22) Date of filing: 09.10.1989
(51) Int. Cl.: A23L 1/305, A23G 3/00

(54) **Proteinaceous composition**
Proteinhaltige Zusammensetzung
Composition protidique

(30) Priority: 11.10.1988 GB 8823804
(43) Date of publication of application: 18.04.1990
(73) Proprietor: Clintec Nutrition Company (an Illinois partnership), Deerfield, Illinois 60015 (US)
(72) Inventor: Ashby, Peter, York (GB); Sayer, Peter J., Gwynedd LL40 (Wales) (GB)
(74) Representative: Pate, Frederick George

(56) References cited:
- EP-A- 0 120 705
- DATABASE WPIL/DERWENT accession no. 87-340211 (48), 1987, Derwent Publications Ltd., London, GB; & SU - A - 1303123 (A. MED. KAZA NUTRITIO) 15.04.1987
- DATABASE WPIL/DERWENT accession no. 87-361051 (51), 1987, Derwent Publications Ltd., London, GB; & SU - A - 1309948 (A. MED. KAZA FOOD SUP.) 15.05.1987

## Description

### Field of the Invention

This invention relates to an edible composition designed to make palatable nutritional or other materials which have an unacceptable taste but which should be taken orally in larger amounts than can be provided in a conventional pharmaceutical dosage form.

### Background of the Invention

Free amino-acids have a strong salty taste and may also have unpleasant "beefy" or bitter flavours. There are a number of clinical disorders which are treated by dietary management, such that most of the patient's protein requirement is provided as free amino-acids. For example, phenylketonurics must not take high protein foods, but instead a food based on a phenylalanine-depleted mixture of free amino-acids. Also sportsmen may choose to ingest a part of their protein intake in the form of free amino-acids.

WPIL/DERWENT Nos. 87-340211 (48) and 87-361051 (51) disclose confectionery products containing more than 2% amino acids, egg white, citric acid, essential oil and sugar. EP-A-0120705 refers to a high protein nutritionally balanced snack obtained by cold extrusion/bar forming equipment.

Traditionally, amino-acids are supplied to patients with disorders such as phenylketonuria, as powders. These may simply be sprinkled on foods or be formulated as flavoured drinks. Sportsmen consume amino-acids in capsules which prevent perception of the unpleasant flavour.

The use of amino-acids in manufactured foods is restricted by at least two factors:
(a) Amino-acids cannot be cooked in the presence of sugars. Maillard reactions cause development of brown colours and off-flavours, and result in rapid destruction of essential amino-acids such as lysine. The nutritional value of the amino-acids is lost.
(b) While proteins can provide structure, e.g. gelatin can set jellies, and milk caseins can firm yoghurt, amino-acids cannot confer structure on a food.

### Summary of the Invention

The present invention is based on the discovery that compositions, which can be formulated as confectionery snacks, can contain more than 2% and, preferably, more than 10% of an unpalatable metabolic intermediate, preferably free amino-acids. A novel composition comprises from 40-80% by weight of a dried or candied fruit, from 3-15% by weight of a gelling agent, a flavour and from 2-25% by weight of the unpalatable material, each weight based upon the weight of the composition. Preferably, the amount of the non-palatable metabolic ingredient is from 10 to 25% by weight.

Compositions of the invention can be simply formed, e.g. to bars of any desired width and depth, by using cold extrusion/bar forming equipment of the type in common use within the confectionery industry. The compositions are microbiologically safe and stable. They do not require cooking.

A particular formulation exemplified below, conveys 5 g of free amino-acid per serving, i.e. a 48 g bar. 5 g of an appropriately formulated amino-acid mixture provides one-sixth of the Recommended Daily Amount (RDA) of protein for a child aged 4-6 years (as specified by the Food and Nutrition Board of the National Research Council of the United States). A formulation can be well-suited to the nutrition of phenylketonuric children.

### Detailed description of the invention

One component of the novel composition is dried fruit or candied fruit. A bland fruit of low protein content such as papaya or melon is preferred. Apricot may be used for apricot-flavoured products. This will usually be the major constituent of the composition, preferably 50 to 75, % by weight.

A second component of the novel composition is a gelling agent. A non-protein gelling agent is required for use with amino-acids. Cold-setting starches or gums or blends of gums and starch may be used. Miragel 463, a cold-setting starch, is particularly favoured. The gelling agent is used in an amount of 3 to 15% by weight which serves its essential structuring function in the product.

The novel composition also comprises a flavour. Freeze-dried fruit of distinctive flavour has been found to be particularly effective in masking the taste of amino-acids. A minor proportion of strongly-flavoured dried/candied fruit may be used to supplement the flavour, as may citric, malic or another acid. When an amino-acid or mixture of amino-acids is used, it is formulated to suit the nutritional purpose of the product. Formulations of various kinds, e.g. for phenylketonurics or people with other disorders of amino-acid metabolism, are well-known and commercially-available. The amino-acid(s) may be used as a powder or may be encapsulated in a hard fat to mask the flavour further and to prolong the life of the amino-acids in the stored product. The amount of the amino-acid component can be 10 to 25% by weight of the composition.

A vitamin mineral pre-mix may be added, if desired. In particular, the product may be supplemented with vitamins and minerals to improve its nutritional quality for people on diets which restrict the range of foods available. Examples of vitamins are vitamin A, thiamin, riboflavin, vitamin B₆, vitamin B₁₂, vitamin C, vitamin D, vitamin E, vitamin K, niacin, folic acid, pantothenic acid and biotin. Examples of minerals are sodium, potassium, calcium, magnesium, iron, zinc, copper, manganese, selenium, chromium, molybdenum, phosphorus, iodine and chloride.

Any supplement can be related to any national Recommended Daily Amounts of vitamins and minerals. A meal replacement which forms part of a diet that includes one or more "normal" meals per day could, in the UK, contain vitamin A, thiamin, riboflavin, vitamin B₁₂, vitamin C, vitamin D, niacin, folic acid, calcium, iron and iodine.

Nutritional requirements for proteins and micro-nutrients change with age. A product of the invention may be in the form of, say, 30-60 g bars or relatively small, e.g. 10-30 g, bars of which a small child might eat three and a young adult 4-5, in order to obtain their daily requirement of the nutritional content.

Any additional ingredients in the novel composition are selected for low protein content, if the product is to be used by people with disorders of protein or amino-acid metabolism. For example, low protein starch-based extrudates may be included, as texturing aids.

The composition should have a water activity of less than 0.65, in order to ensure microbiological safety and stability for the product. A syrup of non-reducing sugars or sugar alcohols may be used to bind the mass, if the other ingredients are not inherently moist. Sugars or sugar alcohols which are non-reducing are used in the centre formulation to minimise Maillard reactions in storage.

The composition may be produced by mixing the ingredients under low shear until it binds together into a coherent combination. Forming extrusion may then take place by means of two contra-rotating, fluted rollers of variable speed. These draw the mass towards a third rotating roller which has a number of grooves cut into it. The dimensions of these grooves match the depth and width of the require snack. The continuous ropes of snack formed on this third roller fall on to a moving conveyor and are guillotined to the correct length. The snack may then be enrobed. If the product is to be used to treat a disorder of protein metabolism, a coating such as plain chocolate is preferred for its low inherent protein content.

The following Examples illustrate the invention.

### Example 1

Apricot-flavoured bars suitable for phenylketonurics were prepared from:

| Ingredient | Amount (kg) |
|---|---|
| Candied (yellow) papaya | 60 |
| Freeze-dried apricots | 14 |
| Miragel* 463 starch | 8 |
| Amino-acids | 14.16 |
| Vitamin mineral pre-mix | 1.87 |
| Natural apricot flavour | 1.0 |
| Malic acid | 0.4 |
| Water | 0.8 |

| | |
|---|---|
| * "Miragel" is a Registered Trade Mark. | |

The following preparative steps were taken:
1) Mince the papaya
2) Dissolve the malic acid in the water
3) Mix the minced papaya, amino-acid mixture and vitamin pre-mix together
4) Add the remainder of the ingredients and mix until a paste is formed
5) Feed the material into a bar former
6) Enrobe the bars in plain chocolate which forms 25% of the finished weight.

### Example 2

Orange-flavoured bars suitable for phenylketonurics were prepared from:

| Ingredient | Amount (kg) |
|---|---|
| Candied (red) papaya | 60 |
| Freeze-dried orange | 14 |
| Miragel* 463 starch | 8 |
| Amino-acid mixture | 16.88 |
| Vitamin pre-mix | 2.28 |
| Whole orange flavour | 0.88 |
| Candied orange peel | 1.6 |

| | |
|---|---|
| * "Miragel" is a Registered Trade Mark. | |

The following preparative steps were taken:
1) Mince the papaya and the orange peel
2) Mix the papaya, amino-acids and vitamin pre-mix. Add the orange flavour and Miragel 463 and, finally, the freeze-dried orange and minced orange peel
3) Mix until a paste is formed
4) Feed the paste into a bar former
5) Enrobe the bars in plain chocolate which forms 25% of the finished weight
Bars weighing 48 g contain approximately 0.75 g protein, 5.0 g amino-acids, 3.7 g fat and 28 g carbohydrate. One-third of the daily requirement of vitamins and trace minerals is provided (in terms of the Acceptable Daily Dietary Intake defined by the Food and Nutritional Board of the National Research Council of the United States).

Each bar is a palatable food, containing less than 50 mg phenylalanine per serving, suitable for phenylketonurics. The necessary components are presented in a form attractive to children who are the group with the most restricted diet.

## Claims

1. An edible composition containing an unpalatable metabolic intermediate comprising a mass of a mixture of from 40% to 80% by weight of a dried or candied fruit, from 2% to 25% by weight of at least one unpalatable metabolic intermediate, from 3% to 15% by weight of a gelling agent and a flavour, each weight based upon the weight of the composition.

2. A composition according to claim 1, which comprises 10 to 25% by weight of said unpalatable metabolic intermediate.

3. A composition according to claim 1, wherein the unpalatable metabolic intermediate is a free amino-acid and the gelling agent is a non-protein gelling agent.

4. A composition according to claim 3, wherein said free amino-acid comprises a phenylalanine-depleted mixture of amino-acids, suitable for the management of phenylketonuria.

5. A composition according to claim 1, wherein said flavor comprises freeze-dried fruit.

6. A composition according to claim 1, which additionally comprises a vitamin and/or mineral supplement.

7. A composition according to claim 1, which is in the form of a bar.

8. A composition according to claim 7, wherein said bar is enrobed in plain chocolate.

9. A method of producing a composition according to claim 1 which comprises mixing the ingredients under low shear until they bind together into a coherent combination.

## Patentansprüche

1. Eine eßbare Zusammensetzung, die ein ungenießbares Stoffwechselzwischenprodukt enthält, das eine Masse aus einer Mischung aus von 40 bis 48 Gew.-% einer getrockneten oder kandierten Frucht, von 2 bis 25 Gew.-% wenigstens eines ungenießbaren Stoffwechselzwischenprodukts, von 3 bis 15 Gew.-% eines Geliermittels sowie ein Aroma umfaßt, wobei jedes Gewicht auf das Gewicht der Zusammensetzung bezogen ist.

2. Zusammensetzung nach Anspruch 1, die 10 bis 25 Gew.-% des genannten ungenießbaren Stoffwechselzwischenprodukts umfassen.

3. Zusammensetzung nach Anspruch 1, bei der das ungenießbare Stoffwechselzwischenprodukt eine freie Aminosäure ist und das Geliermittel ein nicht proteinisches Geliermittel ist.

4. Zusammensetzung nach Anspruch 3, bei der die genannte freie Aminosäure eine an Phenylalanin abgereicherte Mischung von Aminosäuren umfaßt, die für die Behandlung von Phenylketonurie geeignet ist.

5. Zusammensetzung nach Anspruch 1, bei der das genannte Aroma eine gefriergetrocknete Frucht umfaßt.

6. Zusammensetzung nach Anspruch 1, die zusätzlich eine Vitamin- und/oder Mineralstoff-Ergänzung enthält.

7. Zusammensetzung nach Anspruch 1, die in Form eines Riegels vorliegt.

8. Zusammensetzung nach Anspruch 7, bei der der genannte Riegel von einfacher Schokolade umhüllt ist.

9. Verfahren zur Herstellung einer Zusammensetzung nach Anspruch 1, das das Mischen der Bestandteile unter niedrigem Scherbedingungen umfaßt, bis diese zu einer kohärenten Kombination abbinden.

## Revendications

1. Composition comestible contenant un intermédiaire métabolique d'un goût désagréable, comprenant une masse d'un mélange de 40 % à 80 % en poids d'un fruit sec ou confit, de 2 % à 25 % en poids d'au moins un intermédiaire métabolique d'un goût désagréable, de 3 % à 15 % en poids d'un agent gélifiant et d'un aromatisant, chaque poids étant exprimé sur la base du poids de la composition.

2. Composition suivant la revendication 1, qui comprend 10 à 25 % en poids de l'intermédiaire métabolique de goût désagréable.

3. Composition suivant la revendication 1, dans laquelle l'intermédiaire métabolique de goût désagréable est un amino-acide libre et l'agent gélifiant est un agent gélifiant non protéique.

4. Composition suivant la revendication 3, dans laquelle l'amino-acide libre consiste en un mélange d'amino-acides pauvre en phénylalanine, convenant pour le traitement de la phénylcétonurie.

5. Composition suivant la revendication 1, dans laquelle l'agent aromatisant comprend un fruit lyophilisé.

6. Composition suivant la revendication 1, qui comprend en outre un supplément vitaminique et/ou minéral.

7. Composition suivant la revendication 1, qui est sous forme d'une barre.

8. Composition suivant la revendication 7, dans laquelle la barre est enrobée dans du chocolat ordinaire.

9. Composition de production d'une composition suivant la revendication 1, qui comprend le mélange des ingrédients dans des conditions de faible cisaillement jusqu'à ce que ces ingrédients se lient les uns aux autres en formant une association cohérente.
